# EUROPEAN PATENT SPECIFICATION

(11) **EP 2 560 646 B1**
(45) Date of publication and mention of the grant of the patent: **17.12.2014**
(21) Application number: 11716792.4
(22) Date of filing: 21.04.2011
(51) Int. Cl.: A61K 31/451, A61K 31/485, A61P 25/04, A61P 29/02, A61K 31/138, A61K 31/38, A61K 45/06

(54) **COMBINATION OF A SEROTONIN AND NOREPINEPHRINE REUPTAKE INHIBITOR AND AN OPIOID AGONIST FOR THE TREATMENT OF PAIN**
KOMBINATION AUS EINEM SEROTONIN- UND NOREPINEPHRIN-WIEDERAUFNAHMEHEMMER SOWIE EINEM OPIOID ZUR SCHMERZBEHANDLUNG
ASSOCIATION D'UN INHIBITEUR DE RECAPTURE DE SÉROTONINE ET DE NORÉPINEPHRINE ET D'UN AGONISTE OPIOÏDE DANS LE TRAITEMENT DE LA DOULEUR

(30) Priority: 22.04.2010 US 326944 P
(43) Date of publication of application: 27.02.2013
(73) Proprietor: Theravance Biopharma R&D IP, LLC, South San Francisco, CA 94080 (US)
(72) Inventor: MARTIN, William J., San Francisco, California 94114 (US); SHEN, Fei, South San Francisco, California 94080 (US); SMITH, Jacqueline A.M., Redwood City, California 94061 (US)
(74) Representative: Scott, Susan Margaret
(86) International application number: PCT/US2011/033461
(87) International publication number: WO 2011/133790

(56) References cited:
- WO-A1-2010/056939
- US-A1- 2004 019 116
- US-A1- 2004 235 925
- VENTURA VARGAS ET AL: "338 Oxycodone in neuropathic pain: A clinical experience", EUROPEAN JOURNAL OF PAIN, SAUNDERS, LONDON, GB, vol. 11, no. 1, 12 May 2007 (2007-05-12), page 150, XP022076302, ISSN: 1090-3801
- JONES CARRIE K ET AL: "Efficacy of duloxetine, a potent and balanced serotonergic and noradrenergic reuptake inhibitor, in inflammatory and acute pain models in rodents.", THE JOURNAL OF PHARMACOLOGY AND EXPERIMENTAL THERAPEUTICS FEB 2005 LNKD- PUBMED:15494550, vol. 312, no. 2, February 2005 (2005-02), pages 726-732, XP002663521, ISSN: 0022-3565
- GRAY DAVID L ET AL: "Discovery and pharmacological characterization of aryl piperazine and piperidine ethers as dual acting norepinephrine reuptake inhibitors and 5-HT1A partial agonists.", BIOORGANIC & MEDICINAL CHEMISTRY LETTERS 1 DEC 2009 LNKD- PUBMED:19854053, vol. 19, no. 23, 1 December 2009 (2009-12-01), pages 6604-6607, XP002667797, ISSN: 1464-3405

## Description

### BACKGROUND OF THE INVENTION

### FIELD OF THE INVENTION

The present invention is in the field of serotonin and norepinephrine reuptake inhibitors having specified characteristics for the treatment or management of pain, and the use of a specific serotonin and norepinephrine reuptake inhibitor, by itself or in combination with an opioid agonist, for the treatment or management of pain.

The present invention is defined in the appended claims. Subject-matters which are not encompassed by the scope of the claims do not form part of the present invention.

### STATE OF THE ART

Pain is an unpleasant sensory and emotional experience associated with actual or potential tissue damage, or described in terms of such damage (International Association for the Study of Pain (IASP), Pain Terminology). Acute pain, such as that following surgery, usually resolves when the underlying source of the pain is removed or the injury has healed. Chronic pain persists beyond acute pain or beyond the expected time for an injury to heal (American Pain Society. "Pain Control in the Primary Care Setting." 2006:15). Neuropathic pain is pain initiated or caused by a primary lesion or dysfunction in the nervous system. Peripheral neuropathic pain occurs when the lesion or dysfunction affects the peripheral nervous system and central neuropathic pain occurs when the lesion or dysfunction affects the central nervous system (IASP).

Several types of therapeutic agents are currently used to treat pain. Chief among these are opioid agonists such as morphine, oxycodone, methadone, levorphanol and tramadol. The clinical use of morphine, in particular, and other opioid agonists as analgesic agents is limited by safety and tolerability concerns, including respiratory depression, sedation, nausea and vomiting, as well as the potential to develop tolerance, and physical and psychological dependence.

Efforts to achieve a greater therapeutic index with opioids have included development of alternative formulations, e.g. delayed or extended release, and opioid rotation strategies wherein patients rotate through different opioid agonists over the course of treatment. The introduction of patient-controlled analgesia has enabled self-titration of the opioid agonists to optimize the trade off between analgesic efficacy and side effects associated with treatment. However, to date, reduction of opioid consumption yields less analgesia.

Another approach to enhancing the therapeutic index of opioid agonists is combination therapy where the opioid is administered in combination with an agent that treats pain by a different mechanism of action. Medicinal agents that augment endogenous neurotransmitter levels, such as serotonin and norepinephrine reuptake inhibitors (SNRIs) have demonstrated clinical benefit for the treatment of several pain conditions. These agents inhibit the reuptake of both serotonin (5-hydroxytrypamine, 5-HT) and norepinephrine (NE) by binding to the serotonin and norepinephrine transporters (SERT and NET, respectively). More than 20 years ago, desipramine, a tricyclic antidepressant which inhibits norepinephrine and serotonin reuptake was shown to enhance morphine analgesia in post operative dental pain (Levine et al. (1986) Pain 27:45-49). The potential of combination therapy for the treatment of pain remains an active field of study. For example, the interaction between morphine and the SNRI's maprotiline and amitriptyline administered by intrathecal injection in a preclinical rat pain model was recently reported (Pettersen et al. (2009) Anestheia & Analgesia 109:1312-1317).

US20040019116 discloses a SNRI (serotonin and norepinephrine reuptake inhibitor) compound milnacipran which is used for the treatment of pain. This document mentions that milnacipran can be adjunctively administered with morphine.

Despite the early indication of utility of combination therapy and the years of study, no treatment protocol that combines SNRI's and opioid agonists is in common use. Thus, there remains a need to exploit the potential utility of the combination of SNRI's and opioid agonists for the treatment of pain. There also remains a need to identify the pharmacological properties of SNRI's that provide the greatest analgesia when combined with opioid agonists.

### SUMMARY OF THE INVENTION

The present invention provides a serotonin and norepinephrine reuptake inhibitor agent which is 4-[2-(2,4,6-trifluoro-phenoxymethyl)-phenyl]-piperidine or a pharmaceutically acceptable salt thereof, for use in combination with an opioid agonist agent for the treatment of a pain condition in a mammal, characterised in that said serotonin and norephinephrine reuptake inhibitor agent is for use at a dosage of from 2 to 50 mg per day based on a 70 kg human. The invention permits the opioid agonist agent to be used at a dose that is lower than a dose used for treating a pain condition when used alone.

The dual serotonin and norepinephrine reuptake inhibitor 4-[2-(2,4,6-trifluoro-phenoxymethyl)-phenyl]-piperidine (1) and pharmaceutically-acceptable salts thereof 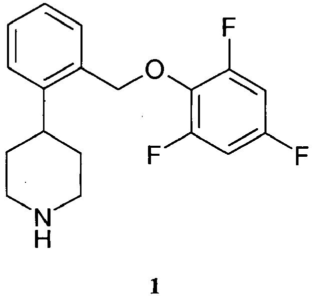 is disclosed in U.S. application serial nos. 12/617,821 and 12/617,838, published 20 May 2010 as US 2010/0125092 A1 and US 2010/0125093 A1, respectively. In particular, the hydrochloride salt of compound 1 is disclosed therein.

Compound 1 has been shown to be a potent inhibitor of NET and SERT activity in cell-based *in vitro* neurotransmitter uptake assays and has demonstrated efficacy *in vivo* in preclinical models of pain. Accordingly, compound 1 is expected to be beneficial for the treatment of pain. Coadministration of compound 1 together with a subefficacious dose of morphine has demonstrated a significant decrease in pain behavior in a rat formalin pain model. Thus, compound 1 has demonstrated a synergistic interaction with morphine for analgesia. In particular, coadministration of compound 1 with an opioid agonist is expected to provide effective analgesia at lower opioid doses, i.e. is expected to be opioid sparing.

The opioid agonist is administered at a dose that is subefficacious for providing analgesia when used alone, i.e. at a dose that is lower than the dose required to achieve a given level of analgesia when used alone. In a specific aspect, in the combination therapy, a subefficacious dose of 4-[2-(2,4,6-trifluoro-phenoxymethyl)-phenyl]-piperidine and a subefficacious dose of the opioid agonist is administered to the patient. In a specific aspect, the pain condition is chronic pain.

Opioid agonists used as analgesic agents for the treatment of pain include morphine, oxycodone, methadone, levorphanol and tramadol. In a specific aspect, the opioid agonist agent is morphine. In another specific aspect, the opioid agonist agent is oxycodone.

The invention may be carried out using a pharmaceutical composition comprising 4-[2-(2,4,6-trifluoro-phenoxymethyl)-phenyl]-piperidine or a pharmaceutically acceptable salt thereof, an opioid agonist agent, and a pharmaceutically-acceptable carrier. In particular aspects, the opioid agonist agent is morphine, or the opioid agonist agent is oxycodone.

### BRIEF DESCRIPTION OF THE DRAWINGS

Various aspects of the present invention are illustrated by reference to the accompanying drawings.

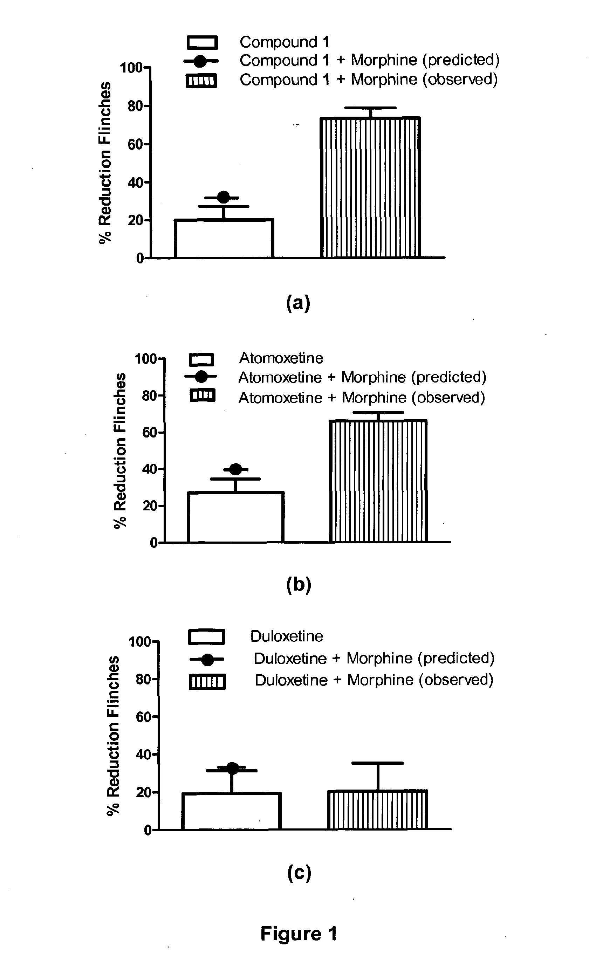

Figure 1 compares the percentage in reduction of flinches normalized to reduction in flinches of vehicle-treated animals in the rat formalin model predicted by adding the separate effects of an SNRI (open bar, first n value) and 1 mg/kg morphine • (n=4) with the effect observed from the combination (striped bar, second n value) for (a) 4-[2-(2,4,6-trifluoro-phenoxymethyl)-phenyl]-piperidine hydrochloride (10 mg/kg) (n=12,13), (b) atomoxetine hydrochloride (10 mg/kg) (n=12,16), and (c) duloxetine hydrochloride (5 mg/kg) (n=5,5), where n is the number of replicates.

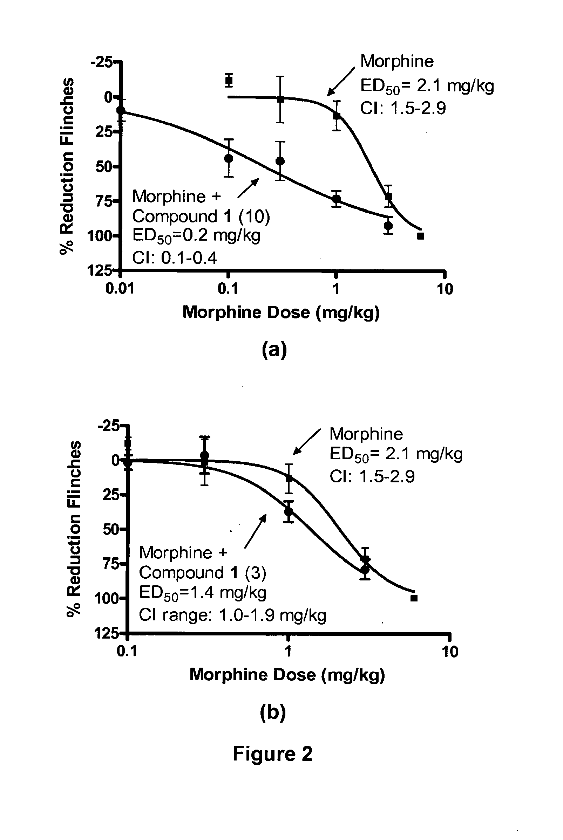

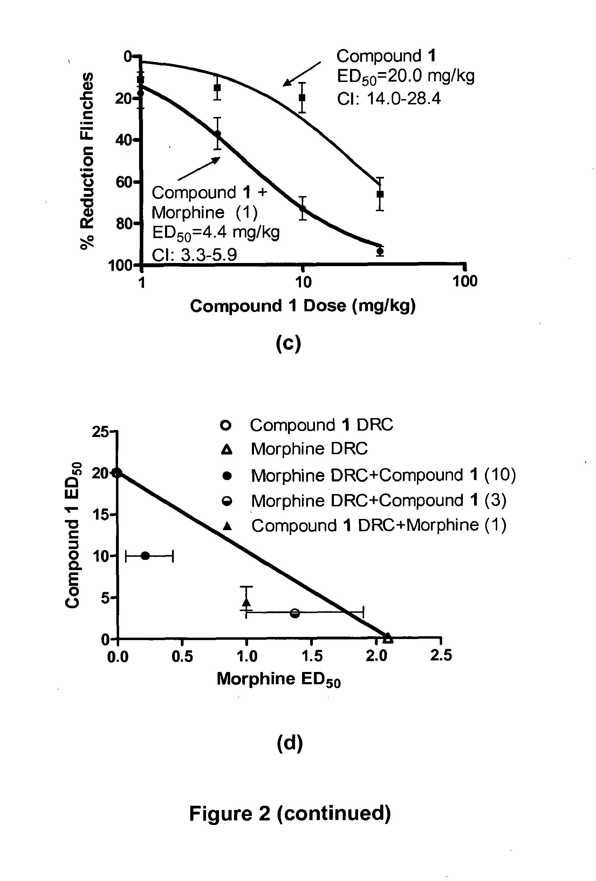

Figure 2 shows results for 4-[2-(2,4,6-trifluoro-phenoxymethyl)-phenyl]-piperidine hydrochloride (1) in the rat formalin model. (a) Dose response curves (DRC) for morphine SC alone (n=4-5) and morphine SC plus 10 mg/kg IP compound 1 (n=6-12) with resulting ED₅₀ values and confidence intervals (CI); (b) Dose response curves for morphine SC alone (n=4-5) and morphine SC plus 3 mg/kg IP compound **1** (n=6-12) with resulting ED₅₀ values and confidence intervals (CI); (c) Dose response curves for compound **1** IP alone (n=13-15), and compound **1** IP plus 1 mg/kg morphine SC (n=6-12) with resulting ED₅₀ values and confidence intervals (CI); (d) Isobolographic analysis comparing ED₅₀ from morphine SC dose response + 10 mg/kg IP compound **1;** ED₅₀ from morphine SC dose response + 3 mg/kg IP compound 1; and ED₅₀ from compound **1** IP dose response + morphine SC 1 mg/kg with line of additivity drawn between ED₅₀ from compound **1** dose response curve and ED₅₀ from morphine dose response curve. (n=n₁-n₂) denotes the range of replicate numbers over the dose response curve.

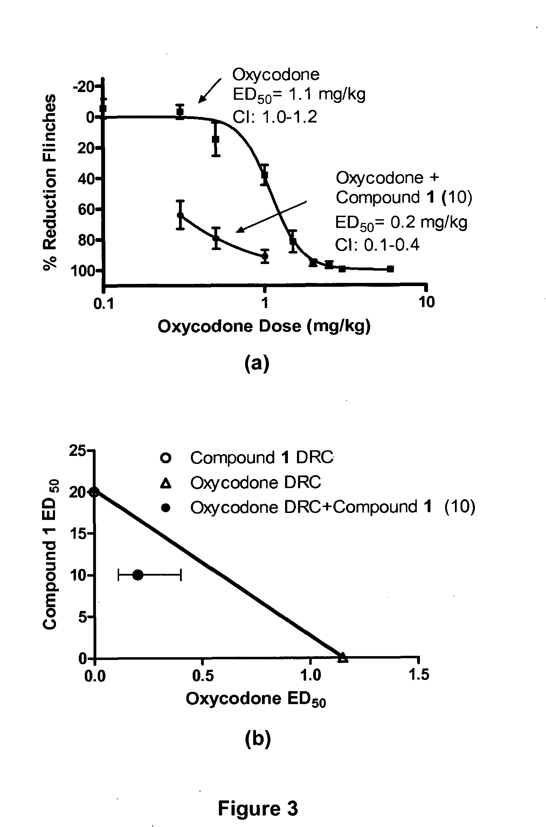

Figure 3 shows results for 4-[2-(2,4,6-trifluoro-phenoxymethyl)-phenyl]-piperidine hydrochloride (1) in the rat formalin model. (a) Dose response curves (DRC) for oxycodone SC alone (n=4-13) and oxycodone SC plus 10 mg/kg IP compound **1** (n=5-6) with resulting ED₅₀ values and confidence intervals (CI); (b) Isobolographic analysis comparing ED₅₀ from oxycodone SC dose response + 10 mg/kg IP compound **1** with line of additivity drawn between ED₅₀ from compound **1** dose response curve and ED₅₀ from oxycodone dose response curve.

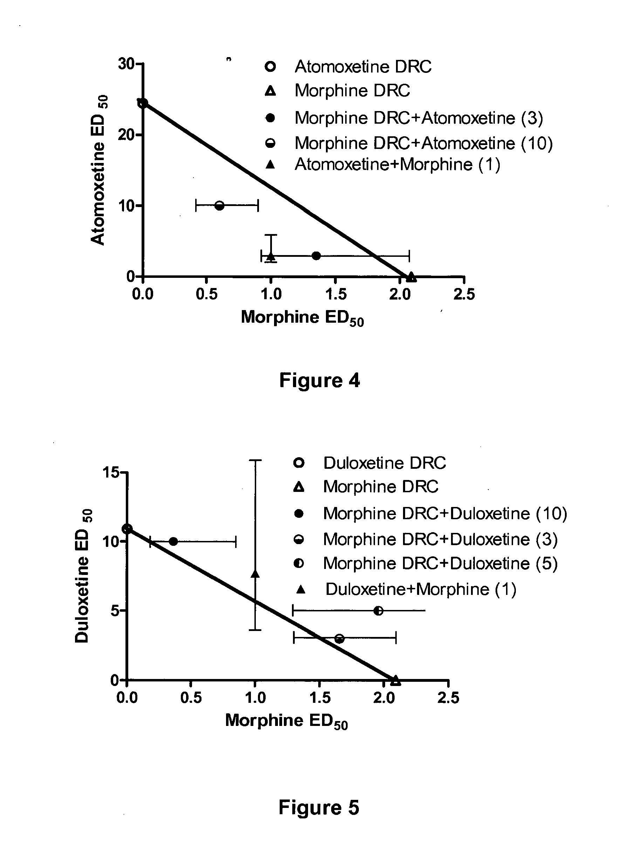

Figure 4 shows isobolographic analysis of the results for atomoxetine in the rat formalin model.
Figure 5 shows isobolographic analysis of the results for duloxetine in the rat formalin model.

### DETAILED DESCRIPTION OF THE INVENTION

### DEFINITIONS

When describing the invention, the following terms have the following meanings unless otherwise indicated. Additionally, as used herein, the singular forms "a," "an" and "the" include the corresponding plural forms unless the context of use clearly dictates otherwise.

The term "therapeutically effective amount" means an amount sufficient to effect treatment when administered to a patient in need thereof, i.e., the amount of drug needed to obtain the desired therapeutic effect. For example, a therapeutically effective amount for treating pain is an amount of compound needed to, for example, reduce, suppress, eliminate or prevent the symptoms of pain or to treat the underlying cause of pain. On the other hand, the term "effective amount" means an amount sufficient to obtain a desired result, which may not necessary be a therapeutic result.

The term "subefficacious amount", or equivalently "subefficacious dose" means an amount or dose lower than a therapeutically effective amount or dose. In the context of treating pain, a "subefficacious dose" is a dose that is lower than the dose required to achieve a given level of analgesia.

The term "treating" or "treatment" as used herein means the treating or treatment of a disease or medical condition (such as pain) in a patient, such as a mammal (particularly a human), that includes one or more of the following: (a) preventing the disease or medical condition from occurring, i.e., prophylactic treatment of a patient; (b) ameliorating the disease or medical condition, i.e., eliminating or causing regression of the disease or medical condition in a patient; (c) suppressing the disease or medical condition, i.e., slowing or arresting the development of the disease or medical condition in a patient; or (d) alleviating the symptoms of the disease or medical condition in a patient.

For example, the term "treating pain" would include managing pain, relieving pain, preventing pain from occurring, controlling pain, ameliorating pain, suppressing pain, and/or alleviating the symptoms of pain. The term "patient" is intended to include those mammals, such as humans, that are in need of treatment or disease prevention, that are presently being treated for disease prevention or treatment of a specific disease or medical condition, as well as test subjects in which compounds of the invention are being evaluated or being used in a assay, for example an animal model.

The term "combination therapy" as used herein means the administration of two or more therapeutic agents as part of a treatment protocol intended to provide beneficial effects from the combined action of the therapeutic agents.

The term "chronic pain" means pain lasting beyond the expected recovery period from an original injury or condition. One example of chronic pain is postherpetic neuralgia where the pain associated with the acute herpes zoster infection persists beyond the infection itself. Clinically, "chronic pain" may be defined as pain lasting more than a given time period, typically about three months.

The term "synergy" or "synergistic effect" as used herein in describing the effect of a combination of two agents means an effect that is greater than the sum of the individual effects. As described below, synergy is demonstrated with statistical significance by a shift in ED₅₀ value, the dose at which a half-maximal effect is observed, with non-overlapping 95 % confidence intervals, and by isobolographic analysis.

The term "compound 1" defined herein as 4-[2-(2,4,6-trifluoro-phenoxymethyl)-phenyl]-piperidine optionally also includes pharmaceutically acceptable salts of 4-[2-(2,4,6-trifluoro-phenoxymethyl)-phenyl]-piperidine, consistent with the context in which the term is used.

The term "morphine" includes all pharmaceutically acceptable salts and administration forms of morphine, including, in particular, morphine sulfate.

The term "oxycodone" includes all pharmaceutically acceptable salts and administration forms of oxycodone, including, in particular oxycodone hydrochloride.

All other terms used herein are intended to have their ordinary meaning as understood by those of ordinary skill in the art to which they pertain.

### DEMONSTRATION OF SYNERGY WITH OPIOID AGONIST IN PRECLINICAL MODEL

The efficacy of the combination of 4-[2-(2,4,6-trifluoro-phenoxymethyl)-phenyl]-piperidine **(1)** and the opioid agonists morphine and oxycodone in relieving pain was evaluated in the rat formalin pain model. The extent to which test compounds inhibit paw-flinching behavior induced by formalin injection was observed.

Compound **1** is about 4-fold selective for NET as determined from the ratio of SERT to NET IC₅₀ values observed *in vitro* in a human recombinant cell-based assay and about 10-fold selective as determined *in vitro* using rat brain synaptosomal preparations. As further described in the following examples, ED₅₀ values, the dose at which a half-maximal effect is observed, were determined from dose-response curves for compound **1** alone, morphine alone, compound **1** in the presence of a fixed dose of morphine, and morphine in the presence of fixed doses of compound **1.** Addition of compound **1** at a dose of 10 mg/kg significantly shifted the morphine dose response curve to lower morphine doses, that is coadministration of compound **1** and morphine allowed comparable efficacy to be achieved at lower doses of morphine. Note the non-overlapping confidence intervals in Fig. 2a. Similarly addition of morphine at 1 mg/kg significantly shifted the compound **1** dose response curve to lower compound **1** doses (Fig. 2c).

The synergistic interaction between compound **1** at 10 mg/kg and morphine is confirmed mathematically by isobolographic analysis which shows co-administration of the two drugs produces an effect that is greater than what would be expected if the effect were merely additive, i.e. the ED₅₀ for the dose response curve for morphine plus compound **1** (10 mg/kg) lies completely to the left of the line of additivity (*See* Example 1 and Fig. 2d). The greater than additive effect can also be illustrated by comparing the sum of the individual effects with the results of coadministration. Single administration of 10 mg/kg of compound **1** inhibited flinching behavior by 20 ± 7 % and administration of 1 mg/kg morphine inhibited flinching behavior by 13 ± 11 %, neither of which is considered statistically significant. As shown in Figure 1a, the 73 ± 6 % inhibition resulting from coadministration of subefficacious doses (10 mg/kg compound **1** and 1 mg/kg morphine) is considerably greater than would be expected if the effect were merely additive.

Similar results were obtained in the rat formalin model for coadministration of compound **1** and oxycodone. Addition of compound 1 at a dose of 10 mg/kg significantly shifted the oxycodone dose response curve to lower oxycodone doses, as shown by the non-overlapping confidence intervals in Fig. 3a. The synergistic interaction between compound 1 at 10 mg/kg and oxycodone is confirmed mathematically by isobolographic analysis, which is illustrated in Fig. 3b.

The relationship between occupancy of serotonin and norepinephrine transporters and observed efficacy was also investigated. Occupancy in the lumbar spinal cord of animals dosed with compound **1** at 10 mg/kg, a dose at which synergy with both morphine and oxycodone was observed, was 81 ± 6 % NET and 46 ± 20 % SERT. Occupancy in animals dosed with compound **1** at 3 mg/kg, a dose at which synergy with morphine (the only opioid tested with 3 mg/kg compound 1) was not observed, was 77± 15 % NET and 23±14 % SERT. These results are consistent with the hypothesis that synergy with opioid agonists requires sufficient occupancy of both norepinephrine and serotonin transporters with NET predominating. Furthermore the observation of greater occupancy at NET than at SERT is consistent with observation of NET-selectivity in *in vitro* assays.

### INVESTIGATIONS OF COMPARISON COMPOUNDS

To further understand the ratio of NET to SERT activity characteristic of an SNRI which exhibits synergy with an opioid agonist, compounds with different profiles were also investigated in the rat formalin model.

Atomoxetine has been shown *in vitro* to be about 20-fold selective for NET in a human-recombinant cell-based assay and about 30-fold selective using rat brain synaptosomal preparations. A 10 mg/kg dose of atomoxetine shifted the morphine dose-response curve toward lower morphine doses. Moreover, addition of morphine at 1 mg/kg also shifted the atomoxetine dose response curve to lower atomoxetine doses. The synergistic interaction between atomoxetine and morphine was confirmed by isobolographic analysis (Fig. 4) and further illustrated in Figure 1b where inhibition resulting from coadministration of subefficacious doses (10 mg/kg atomoxetine and 1 mg/kg morphine) is seen to be significantly greater than the sum of the individual effects.

In contrast, duloxetine, which is about 10-fold selective for SERT in a human-recombinant cell-based assay and about 5-fold selective for SERT in rat brain synaptosomal preparations, did not exhibit a morphine sparing effect. Although single administration of 10 mg/kg of duloxetine provided significant inhibition of flinching behavior, and a 10 mg/kg dose of duloxetine shifted the morphine dose response curve to lower morphine doses, a 1 mg/kg dose of morphine failed to shift the duloxetine dose-response curve. The lack of a morphine sparing effect at 3, 5, or 10 mg/kg of duloxetine was confirmed by isobolographic analysis (Fig. 5) and illustrated graphically in Figure 1c for the combination of subefficacious doses (5 mg/kg of duloxetine and 1 mg/kg morphine), which does not significantly exceed the expected additive effect.

Transporter occupancy in animals dosed with 5 mg/kg duloxetine was about 63 % NET and about 91 % SERT. *(See* Table 5 in Assay 2 below). Synergy with morphine was not observed at any dose with a SERT-selective SNRI. However, when the 5-HT₃ receptor antagonist ondansetron was co-administered with a subefficacious dose of duloxetine (5 mg/kg), a morphine sparing effect was observed. This observation suggests that the SERT-mediated augmentation of 5-HT levels by duloxetine prohibits synergistic interaction between a SERT-selective dual SNRI and morphine.

Compounds with proven analgesic activity in humans exhibit efficacy in the rat formalin model (Le Bars (2001) Pharmacol. Rev. 53:597-652; Vissers et al. (2006) Pharmacology, Biochemistry and Behavior 84:479-486). For example, duloxetine, which demonstrated antinociception at 10 mg/kg in the experiments described here, is approved by the U.S. FDA for the treatment of several chronic pain conditions such as diabetic peripheral neuropathic pain, fibromyalgia, chronic low back pain, and osteoarthritis pain. *(See* also Skljarevski et al. (2009) European Journal of Neurology 16:1041-1048; Chappell et al. (2009) Pain 146:253-260). These results provide a method of predicting whether an SNRI will be expected to provide a synergistic interaction with an opioid agonist for the treatment of pain, the method comprising measuring SERT and NET IC₅₀ values in *in vitro* neurotransmitter uptake assays and determining whether the ratio of SERT to NET IC₅₀ values is between about 2 and about 40, including between about 2 and about 30, between about 4 and about 30, and between about 4 and about 10.

### METHODS OF TREATMENT

Accordingly, the SNRI 4-[2-(2,4,6-trifluoro-phenoxymethyl)-phenyl]-piperidine (1) and combinations of compound 1 and an opioid agonist agent, in particular morphine or oxycodone, are expected to be useful for the treatment of pain conditions, in particular, where the opioid agent is used at a dose that is lower than the dose required for optimal analgesic effect when used alone. Combination therapy is expected to allow the same analgesia to be achieved with lower doses of opioids, thus lessening any adverse effects associated with opioid use. Furthermore, combinations of compound 1 and an opioid agonist agent, in particular morphine or oxycodone, are expected to be useful for the treatment of pain conditions, where each agent is present at a subefficacious dose.

Pain disorders where the combination therapy may find utility include acute pain disorders and chronic pain disorders, including inflammatory pain, and neuropathic pain. More specifically, these include pain associated with or caused by: arthritis; back pain including chronic low back pain; chronic post-surgical pain, cancer, including tumor related pain (e.g., bone pain, headache, facial pain or visceral pain) and pain associated with cancer therapy (e.g., post-radiation syndrome); fibromyalgia; headaches including chronic tension headaches; inflammation associated with polymyalgia, rheumatoid arthritis and osteoarthritis; migraine; neuropathic pain including complex regional pain syndrome; post-operative pain; shoulder pain; central pain syndromes, including post-stroke pain, and pain associated with spinal cord injuries and multiple sclerosis; phantom limb pain; pain associated with Parkinson's disease; visceral pain (e.g., irritable bowel syndrome), diabetic peripheral neuropathy (DPN), HIV-related neuropathy, post-herpetic neuralgia (PHN), and chemotherapy-induced peripheral neuropathy.

When used in combination therapy, compound 1 is either physically mixed with the opioid agonist agent to form a composition containing both agents; or each agent is present in separate and distinct compositions which are administered to the patient simultaneously or sequentially in any order. Combination therapy includes administration of the two agents, when formulated separately, substantially at the same time, as well as administration of each agent at a different time.

For example, compound **1** can be combined with a second therapeutic agent using conventional procedures and equipment to form a composition comprising compound **1** and a second therapeutic agent. Additionally, the therapeutic agents may be combined with a pharmaceutically acceptable carrier to form a pharmaceutical composition comprising compound **1,** a second therapeutic agent, and a pharmaceutically acceptable carrier. In this embodiment, the components of the composition are typically mixed or blended to create a physical mixture. The physical mixture is then administered in a therapeutically effective amount using any of the routes described below.

Alternatively, the therapeutic agents may remain separate and distinct before administration to the patient. In this embodiment, the agents are not physically mixed together before administration but are administered simultaneously or at separate times as separate compositions. Such compositions can be packaged separately or may be packaged together as a kit. The kits comprises a dosage form of compound **1** and a dosage form of the opioid agonist agent. The two therapeutic agents in the kit may be administered by the same route of administration or by different routes of administration. For example, compound **1** may be administered orally while the opioid agonist agent may be administered by any of the conventional routes of administration for such agents, such as intrathecal injection, intravenous injection, subcutaneous injection, or oral tablet, capsule, or liquid.

The amount of compound **1** administered per dose or the total amount administered per day as part of combination therapy may be predetermined or it may be determined on an individual patient basis by taking into consideration numerous factors, including the nature and severity of the patient's condition, the condition being treated, the age, weight, and general health of the patient, the tolerance of the patient to the active agent, the route of administration, pharmacological considerations such as the activity, efficacy, pharmacokinetics and toxicology profiles of the active agent and, in particular, the nature and dosage of the opioid agonist agent. Treatment of a patient suffering from a disease or medical condition (such as a pain condition) can begin with a predetermined dosage or a dosage determined by the treating physician, and will continue for a period of time necessary to prevent, ameliorate, suppress, or alleviate the symptoms of the disease or medical condition. Patients undergoing such treatment will typically be monitored on a routine basis to determine the effectiveness of therapy. For example, in treating neuropathic pain, a measure of the effectiveness of treatment may involve assessment of the patient's quality of life, e.g., improvements in the patient's sleeping patterns, work attendance, ability to exercise and be ambulatory, etc. Pain scales, operating on a point basis, may also be used to help evaluate a patient's pain level. Indicators for the other diseases and conditions described herein, are well-known to those skilled in the art, and are readily available to the treating physician. Continuous monitoring by the physician will insure that the optimal amount of active agents will be administered at any given time, as well as facilitating the determination of the duration of treatment.

Suitable doses of compound **1** for treating a pain condition, when used alone, are expected to range from about 2 to about 50 mg per day, including from about 5 to about 30 mg per day, and from about 7 to about 20 mg per day for an average 70 kg human.

When used as part of combination therapy with an opioid agonist agent, an efficacious dose of compound **1,** as described above, or a subefficacious dose of compound **1,** which is less than the dose required for analgesia when used alone, may be administered.

Agents having opioid agonist activity that may be used in combination therapy according to the present invention include codeine, dihydrocodeine, hydrocodone, hydromorphone, morphine, oxycodone, oxymorphone, tramadol, tapentadol, levorphanol, meperidine, pethidine, methadone, buprenorphine, fentanyl, alfentanil, butrophanol, nalbuphine, and sufentanil. In particular aspects of the invention, the opioid agonist agent is morphine. In other particular aspects of the invention, the opioid agonist agent is oxycodone.

Suitable doses of the opioid agonist will typically be determined by the physician in the light of the relevant circumstances, including the severity of the condition to be treated, the chosen route of administration, the specific agent administered and its relative activity, the age, weight and response of the patient, and particularly, the tolerance of the patient to opioids. Efficacious doses of opioid agonists are known to range from as little as 10 mg/day to as much as 60, 120, or 360 mg/day for a 70 kg human. When used a part of combination therapy, the opioid agonist agent is administered in a therapeutically effective amount, i.e. any amount that produces a therapeutically beneficial effect when co-administered with compound 1, where such amounts are expected to be less than the amount required for optimal monotherapy.

### PHARMACEUTICAL COMPOSITIONS AND FORMULATIONS

The present SNRI compound and the opioid agonist agent, are typically administered to a patient in the form of a pharmaceutical composition or formulation. Such pharmaceutical compositions may be administered to the patient by any acceptable route of administration including, but not limited to, oral, rectal, vaginal, nasal, inhaled, topical (including transdermal) and parenteral modes of administration.

The pharmaceutical compositions typically contain a therapeutically effective amount of the active agents. Those skilled in the art will recognize, however, that a pharmaceutical composition may contain more than a therapeutically effective amount, i.e., bulk compositions, or less than a therapeutically effective amount, i.e., individual unit doses designed for multiple administration to achieve a therapeutically effective amount.

Typically, such pharmaceutical compositions will contain from about 0.1 to about 95% by weight of the active agent; preferably, from about 5 to about 70% by weight; and more preferably from about 10 to about 60% by weight of the active agent.

Any conventional carrier or excipient may be used in the pharmaceutical compositions of the invention. The choice of a particular carrier or excipient, or combinations of carriers or excipients, will depend on the mode of administration being used to treat a particular patient or type of medical condition or disease state. In this regard, the preparation of a suitable pharmaceutical composition for a particular mode of administration is well within the scope of those skilled in the pharmaceutical arts. Additionally, the carriers or excipients used in the pharmaceutical compositions of this invention are commercially-available. By way of further illustration, conventional formulation techniques are described in Remington: The Science and Practice of Pharmacy, 20th Edition, Lippincott Williams & White, Baltimore, Maryland (2000); and H.C. Ansel et al., Pharmaceutical Dosage Forms and Drug Delivery Systems, 7th Edition, Lippincott Williams & White, Baltimore, Maryland (1999).

Representative examples of materials which can serve as pharmaceutically acceptable carriers include the following: sugars, such as lactose, glucose and sucrose; starches, such as corn starch and potato starch; cellulose, such as microcrystalline cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical compositions.

Pharmaceutical compositions are typically prepared by thoroughly and intimately mixing or blending the active agent with a pharmaceutically-acceptable carrier and one or more optional ingredients. The resulting uniformly blended mixture can then be shaped or loaded into tablets, capsules, pills using conventional procedures and equipment.

The pharmaceutical compositions are preferably packaged in a unit dosage form. The term "unit dosage form" refers to a physically discrete unit suitable for dosing a patient, i.e., each unit containing a predetermined quantity of active agent calculated to produce the desired therapeutic effect either alone or in combination with one or more additional units. For example, such unit dosage forms may be capsules, tablets, pills or unit packages suitable for parenteral administration.

In one embodiment, the pharmaceutical compositions of the invention are suitable for oral administration. Suitable pharmaceutical compositions for oral administration may be in the form of capsules, tablets, pills, lozenges, cachets, dragees, powders, granules; or as a solution or a suspension in an aqueous or non-aqueous liquid; or as an oil-in-water or water-in-oil liquid emulsion; or as an elixir or syrup; each containing a predetermined amount of a therapeutic compound as an active ingredient.

When intended for oral administration in a solid dosage form (i.e., as capsules, tablets, pills), the pharmaceutical compositions will typically comprise the active agent and one or more pharmaceutically-acceptable carriers, such as sodium citrate or dicalcium phosphate. Optionally or alternatively, such solid dosage forms may also comprise: fillers or extenders, such as starches, microcrystalline cellulose, lactose, sucrose, glucose, mannitol, and/or silicic acid; binders, such as carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and/or sodium carbonate; solution retarding agents, such as paraffin; absorption accelerators, such as quaternary ammonium compounds; wetting agents, such as cetyl alcohol and/or glycerol monostearate; absorbents, such as kaolin and/or bentonite clay; lubricants, such as talc, calcium stearate, magnesium stearate, solid polyethylene glycols, sodium lauryl sulfate, and/or mixtures thereof; coloring agents; and buffering agents.

Release agents, wetting agents, coating agents, sweetening, flavoring and perfuming agents, preservatives and antioxidants can also be present in the pharmaceutical compositions of the invention. Examples of pharmaceutically-acceptable antioxidants include: water-soluble antioxidants, such as ascorbic acid, cysteine hydrochloride, sodium bisulfate, sodium metabisulfate, sodium sulfite; oil-soluble antioxidants, such as ascorbyl palmitate, butylated hydroxyanisole, butylated hydroxytoluene, lecithin, propyl gallate, alpha-tocopherol; and metal-chelating agents, such as citric acid, ethylenediamine tetraacetic acid, sorbitol, tartaric acid, phosphoric acid. Coating agents for tablets, capsules, pills and like, include those used for enteric coatings, such as cellulose acetate phthalate, polyvinyl acetate phthalate, hydroxypropyl methylcellulose phthalate, methacrylic acid-methacrylic acid ester copolymers, cellulose acetate trimellitate, carboxymethyl ethyl cellulose, hydroxypropyl methyl cellulose acetate succinate. Coating agents also include talc, polyethylene glycol, hypomellose and titanium dioxide.

Pharmaceutical compositions may also be formulated to provide slow or controlled release of the active agent using, by way of example, hydroxypropyl methyl cellulose in varying proportions; or other polymer matrices, liposomes and/or microspheres. In addition, pharmaceutical compositions may optionally contain opacifying agents and may be formulated so that they release the active ingredient only, or preferentially, in a certain portion of the gastrointestinal tract, optionally, in a delayed manner. Examples of embedding compositions which can be used include polymeric substances and waxes. The active agent can also be in micro-encapsulated form, if appropriate, with one or more of the above-described excipients.

Suitable liquid dosage forms for oral administration include, by way of illustration, pharmaceutically-acceptable emulsions, microemulsions, solutions, suspensions, syrups and elixirs. Liquid dosage forms typically comprise the active agent and an inert diluent, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (esp., cottonseed, groundnut, corn, germ, olive, castor and sesame oils), glycerol, tetrahydrofuryl alcohol, polyethylene glycols and fatty acid esters of sorbitan, and mixtures thereof. Suspensions, in addition to the active ingredient, may contain suspending agents such as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

The present active agents can also be administered parenterally (e.g. by intravenous, intrathecal, subcutaneous, intramuscular or intraperitoneal injection). For parenteral administration, the active agent is typically admixed with a suitable vehicle for parenteral administration including, by way of example, sterile aqueous solutions, saline, low molecular weight alcohols such as propylene glycol, polyethylene glycol, vegetable oils, gelatin, fatty acid esters such as ethyl oleate. Parenteral formulations may also contain one or more anti-oxidants, solubilizers, stabilizers, preservatives, wetting agents, emulsifiers, buffering agents, or dispersing agents. These formulations may be rendered sterile by use of a sterile injectable medium, a sterilizing agent, filtration, irradiation, or heat.

Alternatively, the agents are formulated for administration by inhalation. Suitable pharmaceutical compositions for administration by inhalation will typically be in the form of an aerosol or a powder. Such compositions are generally administered using well-known delivery devices, such as a metered-dose inhaler, a dry powder inhaler, a nebulizer or a similar delivery device.

When administered by inhalation using a pressurized container, the pharmaceutical compositions of the invention will typically comprise the active ingredient and a suitable propellant, such as dichlorodifluoromethane, trichlorofluoromethane, dichlorotetrafluoroethane, carbon dioxide or other suitable gas. Additionally, the pharmaceutical composition may be in the form of a capsule or cartridge (made, for example, from gelatin) comprising a compound of the invention and a powder suitable for use in a powder inhaler. Suitable powder bases include, by way of example, lactose or starch.

Finally, active agents can also be administered transdermally using known transdermal delivery systems and excipients. For example, the active agent can be admixed with permeation enhancers, such as propylene glycol, polyethylene glycol monolaurate, azacycloalkan-2-ones and the like, and incorporated into a patch or similar delivery system. Additional excipients including gelling agents, emulsifiers and buffers, may be used in such transdermal compositions if desired.

Representative pharmaceutical compositions useful for the treatment of pain include, but are not limited to, the following examples. Compound **1** is typically supplied as a hydrochloride salt, but it will be understood that any form of the compound (i.e. free base or pharmaceutical salt) that is suitable for the particular mode of administration, can be used in the following pharmaceutical compositions.

### Formulation Example A: Hard Gelatin Capsules for Oral Administration

Compound **1** (20 mg), starch (89 mg), microcrystalline cellulose (89 mg), and magnesium stearate (2 mg) are thoroughly blended and then passed through a No. 45 mesh U.S. sieve. The resulting composition is loaded into a hard gelatin capsule (200 mg of composition per capsule).

### Formulation Example B: Hard Non-Gelatin (HPMC) Capsules for Oral Administration

Compound **1** (10 mg), polyoxyethylene sorbitan monooleate (50 mg), and starch powder (250 mg) are thoroughly blended and then loaded into a hard non-gelatin (HPMC) capsule (310 mg of composition per capsule).

### Formulation Example C: Tablets for Oral Administration

Compound **1** (5 mg), microcrystalline cellulose (400 mg), fumed silicon dioxide (10 mg), and stearic acid (5 mg) are thoroughly blended and then compressed to form tablets (420 mg of composition per tablet).

### Formulation Example D: Tablets for Oral Administration

Compound **1** (10 mg), microcrystalline cellulose (160 mg), lactose monohydrate (20 mg), fumed silicon dioxide (5 mg), and magnesium stearate (5 mg) are thoroughly blended and then compressed to form tablets (200 mg of composition per tablet).

### Formulation Example E: Single-scored Tablets For Oral Administration

Compound **1** (15 mg), cornstarch (50 mg), croscarmellose sodium (25 mg), lactose (120 mg), and magnesium stearate (5 mg) are thoroughly blended and then compressed to form single-scored tablet (215 mg of compositions per tablet).

### Formulation Example F: Suspension for Oral Administration

The following ingredients are thoroughly mixed to form a suspension for oral administration containing 20 mg of active ingredient per 10 mL of suspension: Compound **1** (200 mg), sodium benzoate, sodium citrate, purified water (q.s. to 100 mL).

### Formulation Example G: Injectable Formulation

Compound **1** (20 mg) is blended with 0.1 M sodium citrate buffer solution (15 mL). The pH of the resulting solution is adjusted to pH 6 using 1 N aqueous hydrochloric acid or 1 N aqueous sodium hydroxide. Sterile normal saline in citrate buffer is then added to provide a total volume of 20 mL.

### Formulation Example H: Single-scored Tablets For Oral Administration

Compound 1 (5 mg), morphine sulfate (5 mg), cornstarch (50 mg), microcrystalline cellulose (15 mg), hydroxypropyl cellulose (10 mg) lactose (120 mg), and magnesium stearate (5 mg) are thoroughly blended and then compressed to form single-scored tablet (210 mg of compositions per tablet).

### Formulation Example I: Suspension for Oral Administration

The following ingredients are thoroughly mixed to form a suspension for oral administration containing 5 mg of each agent per 10 mL of suspension: compound **1** (50 mg), oxycodone hydrochloride (50 mg) sodium benzoate, sodium citrate, purified water (q.s. to 100 mL).

### EXAMPLES

The interaction between the SNRI of the invention, 4-[2-(2,4,6-trifluoro-phenoxymethyl)-phenyl]-piperidine **(1)** and the mu opioid agonists, morphine and oxycodone, and also the interaction between the comparison SNRI's atomoxetine, and duloxetine, and morphine was evaluated in the rat formalin model of pain.

### RAT FORMALIN MODEL METHODOLOGY

Male Sprague-Dawley rats (animal body weight range: 150-240 g, Harlan, Indianapolis, IN) were housed in pairs on a 12-h light/dark cycle and were given free access to food and water. All experiments were approved by the Theravance Institutional Animal Care and Use Committee and adhere to the guidelines established by the International Association for the Study of Pain. Compounds were assessed for their ability to inhibit the behavioral response evoked by a 50 µL injection of formalin (5 %) as described previously (Yaksh et al., (2001) J Appl Physiol. 90:2386-402). A metal band was affixed to the left hind paw of rats. Each rat was conditioned to the band for 60 min within a plastic cylinder (15 cm diameter). The number of flinches was counted continuously for 60 min using an automated nociception analyzer (UCSD Anesthesiology Research, San Diego, CA). The antinociceptive period was determined from 15 - 40 min post-formalin injection (phase 2A), corresponding to a persistent pain condition, i.e. a pain condition experienced after the response to the initial stimulus had subsided. Test compounds were prepared in 10 % Tween 20 in distilled water (vehicle) and administered by the intraperitoneal (IP) or subcutaneous (SC) route in a volume of 2 mL/kg

To evaluate the potential opioid-sparing effect, the dose-response of morphine was determined in the absence and presence of compound **1** (3 and 10 mg/kg IP), atomoxetine (3 and 10 mg/kg IP), and duloxetine (3, 5, and 10 mg/kg IP). Dose response curves were also determined for compound **1** and the comparison compounds in the presence of morphine (1 mg/kg SC). In addition, the dose response of oxycodone (SC) was determined in the absence and presence of compound **1** (10 mg/kg IP). Further, duloxetine (5 mg/kg, IP) was co-administered with ondansetron (3 mg/kg, IP) in the absence and presence of morphine (1 mg/kg, SC). All test compounds were administered 30 min prior to the formalin injection.

### DATA ANALYSIS

The percent inhibition was determined by comparing the total number of flinches during phase 2A in concurrently tested vehicle-treated rats according to the following formula: (Vehicle - Treatment) / (Vehicle * 100). Percent inhibition values for each rat at each dose were used to generate ED₅₀ values which were determined by fit to a sigmoidal dose-response (variable slope) curve with minima and maxima constrained to 0 and 100, respectively (GraphPad Prism). Data represent mean ± SEM (standard error of the mean).

To evaluate the potential opioid-sparing effect, ED₅₀ values were determined by generating dose-response curves for morphine, compound **1,** duloxetine, and atomoxetine alone, as well as the dose-response curves for SNRIs in the presence of morphine (1 mg/kg, SC) or the inverse. The effects of coadministration of compound **1** and the opioid agonist oxycodone was also investigated. Potential synergy between the pairs of test compounds was evaluated in one or more of the following ways.
(1) Dose-response curves of opioid agonist + SNRI (or the inverse) were compared. A shift in the ED₅₀ values, with non-overlapping 95% confidence intervals, was deemed a statistically-significant effect.
(2) Isobolographic analysis was used to mathematically evaluate whether co-administration of the two drugs produced an additive, less than additive (antagonistic) or more than additive (synergistic) antinociceptive effect in the rat formalin model. (Tallarida RJ,(2000) Drug Synergism and Dose-effect Data Analysis, Chapman & Hall/CRC, pp 5-10). Briefly, the ED₅₀ of each drug (Drug A and Drug B) alone was plotted on x and y axis respectively, a connected line between two ED₅₀s was drawn (line of additivity). A fixed dose of Drug A was co-administered with a full range of doses of Drug B, to generate a combined ED₅₀ value for this pair. If this combined ED₅₀ (with a confidence interval range) is located to the left of the line of additivity, then the interaction of the two drugs is deemed synergistic.
(3) For illustrative purposes only, the magnitude of antinociception (% reduction in flinches) that would be predicted by adding the % reduction in flinches due to morphine (1.0 mg/kg, SC) treatment to the % reduction in flinches obtained with various doses of an SNRI was compared to the degree of antinociception observed from the combination treatment.

### MATERIALS

Compound 1 hydrochloride was prepared according to the procedures described in U.S. application serial no. 12/617,821, the disclosure of which is incorporated herein by reference. Atomoxetine hydrochloride was purchased from AK Scientific, Inc. (Mountain View, CA) and duloxetine hydrochloride was purchased from Waterstone Technology LLC (Carmel, IN). Morphine and oxycodone were purchased from Sigma-Aldrich (St. Louis, MO). Ondansetron was purchased from Tocris (Ellisville, MO). All test articles were dissolved in 10 % Tween 20 in distilled water.

### EXAMPLE 1

### Combination of Compound 1 and Morphine in the Rat Formalin Model

Dose response curves were determined for compound **1** alone, morphine alone, morphine in the presence of 3 mg/kg or 10 mg/kg of compound **1,** and compound **1** in the presence of 1 mg/kg of morphine. As shown in Figure 2a, morphine administration yielded an ED₅₀ value of 2.1 mg/kg with a confidence interval (CI) of 1.5-2.9 mg/kg. Coadministration of 10 mg/kg compound **1** with morphine significantly shifted the morphine dose response curve to lower morphine doses, providing an ED₅₀ value of 0.2 mg/kg with a non-overlapping CI of 0.1-0.4 mg/kg. Coadministration of 3 mg/kg compound **1** with morphine, however, failed to significantly shift the morphine dose response curve, as shown in Figure 2b. The dose response of compound **1** combined with 1 mg/kg morphine is shown in Figure 2c. Addition of a fixed dose of morphine at a dose below the ED₅₀ value, which did not exhibit a statistically significant effect when administered alone, significantly shifted the dose response curve of compound **1** to lower values, providing an ED₅₀ value (CI) of 4.4 (3.3-5.9) mg/kg for the combination as compared with 20.0 (14.0-28.4) for compound **1** alone. The synergistic interaction between compound **1** and morphine is confirmed by the isobologram of Figure 2d where the ED₅₀ of (0.2 mg/kg) for morphine + compound **1** (10 mg/kg) and of 4.4 mg/kg for compound 1 + morphine (1 mg/kg) lie completely to the left of the line of additivity.

The above experiments, in which observations were made 15-40 minutes post formalin injection, provide a preclinical model of persistent pain, which is considered to approximate a human chronic pain condition. Decreases in pain behavior were also observed during the first 10 minutes post injection (phase 1), a response period believed to reflect acute pain processing. Thus, preclinical data suggest that compound **1** and the combination of compound **1** and morphine also provide relief of acute pain conditions, such as post-operative pain.

### EXAMPLE 2

### Combination of Compound 1 and Oxycodone in the Rat Formalin Model

Dose response curves were determined for oxycodone alone and oxycodone in the presence of 10 mg/kg of compound **1.** As shown in Figure 3a, oxycodone administration yielded an ED₅₀ value of 1.1 mg/kg with a confidence interval (CI) of 1.0-1.2 mg/kg. Coadministration of 10 mg/kg compound **1** with oxycodone significantly shifted the oxycodone dose response curve to lower oxycodone doses, providing an ED₅₀ value of 0.2 mg/kg with a non-overlapping CI of 0.1-0.4 mg/kg. The synergistic interaction between compound **1** and oxycodone is confirmed by the isobologram of Figure 3d where the ED₅₀ of (0.2 mg/kg) for oxycodone + compound **1** (10 mg/kg), including error bars, lies to the left of the line of additivity.

### EXAMPLE 3

### Study of Comparison SNRI's in the Rat Formalin Model

The effects of atomoxetine at 3 and 10 mg/kg and of duloxetine at 3, 5, and 10 mg/kg together with morphine are summarized in the following tables. Addition of atomoxetine at 10 mg/kg to morphine provided a lower ED₅₀ value with non-overlapping confidence intervals (Table 1). Coadministration of 1 mg/kg morphine also significantly lowered the atomoxetine ED₅₀ value (Table 2). The synergistic interaction between atomoxetine and morphine is confirmed by the isobologram of Figure 4 where the ED₅₀ of (0.6 mg/kg) for morphine + atomoxetine (10 mg/kg) and of 3.0 mg/kg for atomoxetine + morphine (1 mg/kg), both including error bars, lie to the left of the line of additivity.

While addition of duloxetine at 10 mg/kg to morphine provided a lower ED₅₀ value with non-overlapping confidence intervals (Table 1), the addition of 1 mg/kg morphine to duloxetine failed to shift the duloxetine dose response curve to lower duloxetine doses, i.e. the ED₅₀ values in Table 2 for duloxetine alone and duloxetine + 1 mg/kg morphine have overlapping confidence intervals. The isobologram of Figure 5 shows no combination dose of duloxetine and morphine lies to the left of the line of additivity. Duloxetine did not exhibit synergy with morphine.

**Table 1: Morphine Dose Response**

| | ED₅₀ (Confidence Interval) (mg/kg)* |
|---|---|
| Morphine | 2.1 (1.5 - 2.9) (n=4-5) |
| Morphine + 3 mg/kg Atomoxetine | 1.4 (0.9 - 2.1) (n=6-11) |
| Morphine + 10 mg/kg Atomoxetine | 0.6 (0.4 - 0.8) (n=6-17) |
| Morphine + 3 mg/kg Duloxetine | 1.7 (1.3 - 2.1) (n=6-12) |
| Morphine + 5 mg/kg Duloxetine | 2.0 (1.3 - 3.0) (n=5-6) |
| Morphine + 10 mg/kg Duloxetine | 0.4 (0.2 - 0.8) (n=5-11) |

**Table 2: SNRI Dose Response**

| | ED₅₀ (Confidence Interval) (mg/kg)* |
|---|---|
| Atomoxetine | 24.4 (14.5 - 41.1) (n=6-12) |
| Atomoxetine + 1 mg/kg Morphine | 3.0 (1.6 - 5.7) (n=6-11) |
| Duloxetine | 10.9 (7.8 - 15.3) (n=5-10) |
| Duloxetine + 1 mg/kg Morphine | 10.3 (3.8 - 27.8) (n=5-11) |

| | |
|---|---|
| * (n=n₁-n₂) is the range of replicate numbers over the dose response curve | |

### ASSAY 1

### In Vitro SERT and NET Neurotransmitter Uptake Assays

Neurotransmitter uptake assays were used to measure inhibition of ³H-serotonin (³H-5-HT) and ³H-norepinephrine (³H-NE) uptake into cells expressing the respective human recombinant transporter (hSERT or hNET) and into rat brain synaptosomal preparations in order to determine the pIC₅₀ values of test compounds at the human and rat monoamine transporters, respectively.

### Cell Culture

HEK-293 derived cell lines stably-transfected with hSERT or, hNET, respectively, were grown in Dulbecco's modified Eagles Medium (DMEM) supplemented with 10% dialyzed FBS (for hSERT) or FBS (for hNET), 100 µg/ml penicillin, 100 µg/ml streptomycin, 2 mM L-glutamine and 250 µg/ml of the aminoglycoside antibiotic G418 in a 5% CO₂ humidified incubator at 37°C. When cultures reached 50-80% confluence, the cells were washed thoroughly in PBS (without Ca²⁺ and Mg²⁺) and lifted with 5 mM EDTA in PBS. Cells were harvested by centrifugation at 1100 rpm for 5 minutes, the supernatant was discarded, and the cell pellet resuspended, by gentle trituration, in room temperature Krebs-Ringer bicarbonate buffer containing HEPES (10 mM), CaCl₂ (2.2 mM), ascorbic acid (200 µM) and pargyline (200 µM), pH 7.4. The final concentration of cells in the cell suspension was 7.5 x 10⁴ cells/ml for hSERT and 12.5 x 10⁴ cells/ml for hNET.

### Synaptosome Preparation

Rats were euthanized by decapitation. Rat freshly dissected cortical tissue pieces were suspended in ice-cold Sucrose Buffer at a ratio of 100 mg wet weight of tissue per 1 mL of buffer. The tissues were homogenized using a pre-chilled glass dounce homogenizer for 20 strokes. The homogenate was centrifuged at 1,000 x g (10 min at 4 °C). The pellet was discarded and the resulting supernatant was centrifuged at 10,000 x g (20 min at 4 °C) to obtain the crude synaptosomal pellet. Synaptosomes were resuspended in Sucrose Buffer and the protein concentration was determined by the method of Bradford (Bradford (1976) Analytical Biochemistry 72:24854) and used immediately following preparation.

### Neurotransmitter Uptake Assays

Neurotransmitter uptake assays were performed in a 96-well assay plate in a total volume of 400 µL containing 1.5 x 10⁴ and 2.5 x 10⁴ cells, for SERT and NET, respectively or 10 µg cortical synaptosomal protein in assay buffer (Krebs-Ringer bicarbonate buffer containing HEPES (10 mM), CaCl₂ (2.2 mM), ascorbic acid (200 µM) and pargyline (200 µM), pH 7.4). Inhibition assays for determination of pIC₅₀ values of test compounds were conducted with 11 different concentrations, ranging from 10 pM to 100 µM. Stock solutions (10 mM in DMSO) of test compound were prepared and serial dilutions prepared using 50 mM Tris-HCl, 120 mM NaCl, 5mM KCl, pH 7.4, 0.1% BSA, 400 µM ascorbic acid. Test compounds were incubated for 30 minutes at 37°C with the respective cells or synaptosomes, prior to addition of radiolabeled neurotransmitter, ³H-5-HT (20 nM final concentration), or ³H-NE (40 nM final concentration). Non-specific neurotransmitter uptake was determined in the presence of 2.5 µM duloxetine or 2.5 µM desipramine (each in Dilution Buffer) for the SERT or NET assays, respectively.

Following a 10 minute incubation for cells (6 minute incubation for rat synaptosomes) at 37°C with radioligand, the reactions were terminated by rapid filtration over a 96-well UniFilter GF/B plate, pretreated with 1% BSA, and washed 6 times with 650 µl wash buffer (ice cold PBS). Plates were dried, 35-45 µl of MicroScint™-20 (Perkin Elmer) added and incorporated radioactivity quantitated via liquid scintillation counting. Inhibition curves were analyzed using the GraphPad Prism Software package (GraphPad Software, Inc., San Diego, CA). IC₅₀ values generated from concentration response curves using the Sigmoidal Dose Response (variable slope) algorithm in Prism GraphPad are reported below in Table 3 as the negative decadic logarithm of the IC₅₀ values, pIC₅₀.

**Table 3: In Vitro SERT and NET Neurotransmitter Uptake**

| | HEK-293 cells | | | Rat brain synaptosomes | | |
|---|---|---|---|---|---|---|
| | SERT pIC₅₀ | NET pIC₅₀ | NET selectivity ^{(a)} | SERT pIC₅₀ | NET pIC₅₀ | NET selectivity ^{(a)} |
| Compound **1** | 8.0 | 8.6 | 4 | 7.9 | 8.9 | 10 |
| Atomoxetine | 7.3 | 8.5 | 20 | 7.1 | 8.6 | 30 |
| Duloxetine | 9.2 | 8.1 | 0.1 | 9.1 | 8.4 | 0.2 |

| | | | | | | |
|---|---|---|---|---|---|---|
| (a) NET selectivity was calculated as 10**(NET pIC₅₀ - SERT pIC₅₀) | | | | | | |

### ASSAY 2

### Ex Vivo SERT and NET Transporter Occupancy Studies

SERT and NET transporter occupancy was determined in cortical or lumbar spinal cord homogenates prepared from rats administered compound **1** or duloxetine by the intraperitoneal (IP) route. To monitor SERT and NET transporter occupancy, an *ex vivo* occupancy assay was used in which the initial rate of association of a SERT- or NET-selective radioligand, respectively, to the tissue homogenates was monitored. The initial rate of association of radioligand is directly proportional to the level of free (unoccupied) transporter.

### Tissue Harvest

Rats were euthanized 1.25 hours post compound administration by decapitation. Following decapitation, the brain was removed and placed on to a clean metal block, on ice. A parasagittal cut was made through the entire brain, around 4 mm lateral to the midline. The lateral cortex pieces were discarded. A midsagittal cut was made through the entire length of the brain. The block of brain corresponding to each hemisphere was then placed on to the respective lateral cut surface, such that the midsagittal surface was uppermost. A single cut was made parallel to, and about 1 mm dorsal to, the corpus callosum to remove a cortex piece, which was frozen on dry ice prior to storage at -80°C. The spinal cord was harvested by hydraulic extrusion with PBS. An 18 gauge needle was inserted into the spinal column near the iliac crest and the buffer injected into the spinal column with the resultant extrusion of the spinal cord, intact. The lumbar spinal cord (approximately 1 - 1.5 cm in length) was rapidly dissected and frozen on dry ice prior to storage at -80°C.

The frozen tissue pieces were weighed and kept on dry ice prior to homogenization. Spinal cord tissue was homogenized in Assay Buffer (0.154 mL per 100 mg frozen tissue) with a Polytron homogenizer (Model: PT 2100; setting 19 for 10 sec). The crude homogenates were prepared immediately prior to initiation of the *ex vivo* binding assay.

### Radioligand binding assays

Radioligand binding assays were performed in 96-well polypropylene assay plates in a total assay volume of 200 µL containing ∼25 µg of membrane protein. In all cases the final assay buffer was 50 mM Tris-HCl, 120 mM NaCl, 5 mM KCl, 0.025% BSA, 100 µM Ascorbic Acid, pH 7.4.

Total and non-specific binding was determined at a single concentration of radioligand (in parentheses): [³H]-citalopram (3 nM) and [³H]-nisoxetine (5 nM) at SERT and NET, respectively. Non-specific binding for [³H]-citalopram and [³H]-nisoxetine was determined in the presence of 1 µM duloxetine or 1 µM desipramine, respectively. The *ex vivo* binding assay was initiated by the addition of cortical or spinal cord homogenates at six time points to generate a time course with 0.4, 1.0, 1.5, 2.0, 2.5, and 3 min incubation times for SERT or 0.25, 0.5, 1.25, 1.75, and 2.25 min incubation times for NET. Reactions were terminated by rapid filtration over 96-well GF/B glass fiber filter plates (Packard BioScience Co., Meriden, CT) presoaked in 0.3% polyethyleneimine. Filter plates were washed six times with wash buffer (ice-cold 50 mM Tris-HCl, 0.9% NaCl, at 4°C) to remove unbound radioactivity. Plates were dried, 35 - 45 µL Microscint-20 liquid scintillation fluid (Packard BioScience Co., Meriden, CT) was added to each well, and plates were counted in a Packard Topcount liquid scintillation counter (Packard BioScience Co., Meriden, CT).

### Data Analysis

Specific binding was calculated using total (in the absence of test compound) and non-specific (determined as described above) binding from the same plate as follows: specific binding = value - non-specific binding. Data then were analyzed by linear regression analysis with the GraphPad Prism Software package (GraphPad Software, Inc., San Diego, CA). The initial rates of association of [³H]-citalopram or [³H]-nisoxetine, equal to the slope of the straight line, were determined by fitting data points, specific binding (cpm) versus time (min). The average of the initial rates of [³H]-citalopram or [³H]-nisoxetine association from vehicle-dosed animals was calculated. The % NET or SERT transporter occupancy for animals dosed with test compound is computed using the following equation: % transporter occupancy = 100 x (1- [(initial rate association for compound-dosed animal) / (average initial rates of association for vehicle-dosed animals)]). Occupancies in spinal cord after IP administration of compound **1** are reported below in Table 4

**Table 4: Compound 1 Ex Vivo SERT and NET Transporter Occupancy**

| Compound **1** Dose (mg/kg IP) | rat SERT % Occupancy* | rat NET % Occupancy* |
|---|---|---|
| 3 | 23 ±14 | 77 ± 15 |
| 10 | 46 ± 20 | 81 ± 6 |

| | | |
|---|---|---|
| * ± Standard Error | | |

Occupancies in cortex after IP administration of duloxetine are reported below in Table 5. Occupancy for a dose of 5 mg/kg is expected to be about the average of the 3 mg/kg and 10 mg/kg values. In other studies, comparable values have been observed for spinal cord and cortex occupancies.

**Table 5: Duloxetine Ex Vivo SERT and NET Transporter Occupancy**

| Duloxetine Dose (mg/kg IP) | rat SERT % Occupancy* | rat NET % Occupancy* |
|---|---|---|
| 3 | 86 ± 3 | 52 ± 5 |
| 10 | 96 ± 5 | 73 ± 5 |

| | | |
|---|---|---|
| * ± Standard Error | | |

Occupancies in cortex after IP administration of atomoxetine are reported below in Table 6.

**Table 6: Atomoxetine Ex Vivo SERT and NET Transporter Occupancy**

| Atomoxetine Dose (mg/kg IP) | rat SERT % Occupancy* | rat NET % Occupancy* |
|---|---|---|
| 3 | 35 ± 9 | 67 ± 10 |
| 10 | 64 ± 5 | 84 ± 3 |

| | | |
|---|---|---|
| * ± Standard Error | | |

### ASSAY 3

### Pharmacokinetic Studies

Plasma concentrations in rats of SNRI's administered alone or in combination with an opioid agonist were measured to assess the potential for drug-drug interaction.

Compound **1** (3 mg/kg), duloxetine (10 mg/kg), or atomoxetine (10 mg/kg) were administered IP in combination with morphine (3 mg/kg, SC) or with vehicle (SC). Plasma concentrations of SNRI were equivalent in rats which received compound **1,** atomoxetine, or duloxetine alone or in combination with morphine. Compound **1** (10 mg/kg, IP) was also administered in combination with oxycodone (3 mg/kg, SC) Plasma concentrations of both agents were equivalent in rats which received compound **1** or oxycodone alone, or the combination. These data suggest that evidence for enhanced analgesic efficacy in the formalin assay is unlikely to be a function of an increase in plasma exposure of opioid agonist or of SNRI's upon coadministration, i.e. the observed pharmacological effect cannot be attributed to drug-drug interactions.

## Claims

1. A serotonin and norepinephrine reuptake inhibitor agent which is 4-[2-(2,4,6-trifluoro-phenoxymethyl)-phenyl]-piperidine or a pharmaceutically acceptable salt thereof, for use in combination with an opioid agonist agent for the treatment of a pain condition in a mammal, **characterised in that** said serotonin and norephinephrine reuptake inhibitor agent is for use at a dosage of from 2 to 50 mg per day based on a 70 kg human.

2. The inhibitor agent for use of Claim 1 wherein the inhibitor agent is 4-[2-(2,4,6-trifluoro-phenoxymethyl)-phenyl]-piperidine hydrochloride.

3. The inhibitor agent for use of either Claim 1 or Claim 2 wherein the pain condition is chronic pain.

4. The inhibitor agent for use of any one of Claims 1 to 3 wherein the mammal is a human.

5. The inhibitor agent for use of any one of Claims 1 to 4 wherein the opioid agonist agent is morphine.

6. The inhibitor agent for use of any one of Claims 1 to 4 wherein the opioid agonist agent is oxycodone.

7. The inhibitor agent for use of any one of Claims 1 to 6 wherein the inhibitor agent and the opioid agonist agent are administered by different routes of administration.

## Patentansprüche

1. Ein Serotonin- und Norepinephrin-Wiederaufnahmehemmer, der 4-[2-(2,4,6-Trifluorphenoxymethyl)phenyl]piperidin oder ein pharmazeutisch annehmbares Salz davon ist, zur Verwendung in Kombination mit einem Opioidagonisten zur Behandlung eines Schmerzzustandes bei einem Säuger, **dadurch gekennzeichnet, dass** der Serotonin- und Norepinephrin-Wiederaufnahmehemmer zur Verwendung in einer Dosis von 2 bis 50 mg pro Tag, bezogen auf einen Menschen mit 70 kg, dient.

2. Der Hemmer zur Verwendung gemäß Anspruch 1, wobei der Hemmer 4-[2-(2,4,6-Trifluorphenoxymethyl)phenyl]piperidin-Hydrochlorid ist.

3. Der Hemmer zur Verwendung nach Anspruch 1 oder Anspruch 2, wobei der Schmerzzustand chronischer Schmerz ist.

4. Der Hemmer zur Verwendung nach einem der Ansprüche 1 bis 3, wobei der Säuger ein Mensch ist.

5. Der Hemmer zur Verwendung nach einem der Ansprüche 1 bis 4, wobei der Opioidagonist Morphin ist.

6. Der Hemmer zur Verwendung nach einem der Ansprüche 1 bis 4, wobei der Opioidagonist Oxycodon ist.

7. Der Hemmer zur Verwendung nach einem der Ansprüche 1 bis 6, wobei der Hemmer und der Opioidagonist auf unterschiedlichen Verabreichungswegen verabreicht werden.

## Revendications

1. Agent inhibiteur de recapture de sérotonine et de norépinéphrine qui est le 4-[2-(2,4,6-trifluoro-phénoxyméthyle)-phényle]-pipéridine ou un sel de celui-ci acceptable sur le plan pharmaceutique, à utiliser en combinaison avec un agent agoniste opioïde pour le traitement d'une condition de douleur chez un mammifère, **caractérisé en ce que** ledit agent inhibiteur de recapture de sérotonine et de norépinéphrine doit être utilisé avec un dosage de 2 mg à 50 mg par jour calculé pour un être humain pesant 70 kg.

2. Agent inhibiteur à utiliser selon la revendication 1, dans lequel l'agent inhibiteur est l'hydrochlorure de 4-[2-(2,4,6-trifluoro-phénoxyméthyle)-phényle]-pipéridine.

3. Agent inhibiteur à utiliser selon la revendication 1 ou la revendication 2, dans lequel la condition de douleur est une douleur chronique.

4. Agent inhibiteur à utiliser selon l'une quelconque des revendications 1 à 3, dans lequel le mammifère est un être humain.

5. Agent inhibiteur à utiliser selon l'une quelconque des revendications 1 à 4, dans lequel l'agent agoniste opioïde est la morphine.

6. Agent inhibiteur à utiliser selon l'une quelconque des revendications 1 à 4, dans lequel l'agent agoniste opioïde est l'oxycodone.

7. Agent inhibiteur à utiliser selon l'une quelconque des revendications 1 à 6, dans lequel l'agent inhibiteur et l'agent agoniste opioïde sont administrés par différentes voies d'administration.
